# EUROPEAN PATENT SPECIFICATION

(11) **EP 3 626 815 B1**
(45) Date of publication and mention of the grant of the patent: **07.02.2024**
(21) Application number: 17909797.7
(22) Date of filing: 26.05.2017
(51) Int. Cl.: C12N 1/14, A01H 17/00, A01N 25/22, C12R 1/645

(54) **STRAIN OF MICROORGANISM CLONOSTACHYS ROSEA F. CATENULATA AS A BIOFUNGICIDE, PLANT GROWTH STIMULANT AND METABOLITE PRODUCER FOR AGRICULTURAL USE**
STAMM DES MIKROORGANISMUS CLONOSTACHYS ROSEA F. CATENULATA ALS BIOFUNGIZID, PFLANZENWACHSTUMSSTIMULANS UND METABOLITERZEUGER ZUR ANWENDUNG IN DER LANDWIRTSCHAFT
SOUCHE DE MICRO-ORGANISME CLONOSTACHYS ROSEA F. CATENULATA UTILISÉE COMME BIOFONGICIDE, STIMULATEUR DE CROISSANCE DE PLANTES ET PRODUCTEUR DE MÉTABOLITES À USAGE AGRICOLE

(30) Priority: 18.05.2017 RU 2017117282
(43) Date of publication of application: 25.03.2020
(73) Proprietor: Obshchestvo S Ogranichennoy Otvetsvennostyu "Ekogen", St.Petersburg 196608 (RU)
(72) Inventor: GLOBUS, Galina Alekseyevna, St.Petersburg 195427 (RU); GOLUBEV, Andrey Sergeyevich, St.Petersburg 196608 (RU); BROVTSEV, Mikhail Anatolyevich, St.Petersburg 196600 (RU); GADABORSHEVA, Svetlana Aleksandrovna, St.Petersburg 196600 (RU)
(74) Representative: Lapienis, Juozas
(86) International application number: PCT/RU2017/000357
(87) International publication number: WO 2018/212673

(56) References cited:
- WO-A1-2007/107000
- WO-A1-2015/035504
- WO-A2-2011/117271
- RU-C1- 2 415 917
- RU-C2- 2 154 381
- CHATTERTON S ET AL: "Colonization of cucumber plants by the biocontrol fungus Clonostachys rosea f. catenulata", BIOLOGICAL CONTROL, SAN DIEGO, CA, US, vol. 46, no. 2, 1 August 2008 (2008-08-01) , pages 267-278, XP022796123, ISSN: 1049-9644, DOI: 10.1016/J.BIOCONTROL.2008.02.007 [retrieved on 2008-02-20]
- SUN ZHAN-BIN ET AL: "A Perilipin Gene from Clonostachys rosea f. Catenulata HL-1-1 Is Related to Sclerotial Parasitism", INT. J. MOL. SCI., vol. 16, no. 12, 1 December 2015 (2015-12-01), pages 5347-5362, XP055774237, ISSN: 1661-6596, DOI: 10.3390/ijms16035347

## Description

The invention relates to biotechnology and agricultural microbiology, more particularly to producing bacterial strains with fungicidal, antibacterial and growth-stimulating properties. A strain VKPM-F 1324 of Clonostachys rosea f. catenulata was produced. This strain demonstrates fungicidal properties and growth-stimulating activity and can be used as a crop protection product against various diseases caused by fungal and bacterial phytopathogens. Moreover, the strain is a producer of valuable metabolites, such as amino acids and phytohormones, which are used in agriculture. The invention, by virtue of the properties thereof, makes it possible to increase crop yield.

On the one hand, Clonostachys rosea f. catenulata microorganisms is known as a destructor of polyvinyl alcohol. The prior art document RU 2415915 04.04.2011 C1, which describes the strain Clonostachys rosea f. catenulata (J.C.Gilman et E.VAbbott) Schroers VKPM F-991, used as a biodestructor of polyvinyl alcohol.

On the other hand, it is known that the microorganisms of the species Clonostachys rosea f. catenulata is used in agriculture. It is known to use to improve plant growth, for example, in compositions containing, in addition to the microorganism, also mulch (see WO 2014076663 05.22.2014 A1).

Fungicidal mixtures based on azolopyrimidinylamines are known in the art, in which a strain or cell-free extract and / or a mutant of this strain or extract having all the distinctive characteristics of the corresponding strain or extract of Clonostachys rosea f catenulata are used as antifungal agents of biological control, plant bioactivators. (WO 2011/117271 09/29/2011).

The prior art also knows the use of other species of this microorganism. Thus, the known strain Clonostachys rosea f. rosea, IDAC 040913-01, which suppresses or controls a disease or pathogen that affects leaves, flowers, fruits and / or roots of a plant (WO 2015/035504, 03/19/2015). This document also describes a composition that includes a selected culture of this strain, a method of treating a plant with a biological protection agent, including contacting the plant with a selected culture, spores of a fungus or a specified composition, a method of reducing spoilage of plant material, where the method includes contacting the plant material with a selected culture, spores mushroom or composition.

Strain Clonostachys solani f. nigrovirens (van Beyma) Schroers VKPM F-990 is known as a biodestructor of thermoplastic polyurethane and acrylic acid-based latex (RU 2415917 04.04.2011 C1).

The purpose of the present invention is to obtain a highly efficient strain for use in agriculture to protect agricultural plants from various diseases caused by phytopathogenic fungi and bacteria, to accelerate the growth and development of plants, to increase yield and product quality, as well as to obtain valuable agricultural products, metabolites, such as amino acids and phytohormones.

The authors of the present invention were isolated strain Clonostachys rosea f. catenulate deposited in the All-Russian Collection of Industrial Microorganisms under the number VKPM-F1324.

The strain isolated from the rhizosphere of cotton in the Tashkent region.

### Cultural and morphological characteristics of the strain:

Colonies on wort agar are initially white, pubescent, eventually acquiring a gray-greenish color with concentric zonality, reverse light yellow. Conidiophores along the periphery of a colony of verticylloid type, with widely divergent fialida, in the center dominated by conidiophores of penicilloid type with more densely squeezed fialida, two-and three-tier, conidia oval) in long chains, mucous.

Grows in aerobic conditions. Good growth at 10 ... 30 ° C, grows over a wide range of pH from 3.0 to 9.

The strain is characterized by good growth and sporulation when grown on agar media - wort agar, potato-sucrose medium, Czapek's medium, starch-soybean, medium from bran decoction.

Sporulation starts on the fifth day of growth at 24 ... 26 ° C.

Linear growth of the colony on agar media:
On the wort agar medium on Petri dishes - on the 10th day of growth - 50 mm in size, on the 20th day - 80 mm.
On potato agar on the 14th day of growth - 45 mm, on the 20th day 90 mm.
On the agar medium of čapek with sucrose on the 12th day of growth - 45 mm, on the 20th day - 85 mm.

During deep cultivation on mineral and organic media, the strain grows in the form of a homogeneous mass of beige or green color.

Colonies on Chapek's agar are white, open, fluffy, with a conidy zone in the center of the colony, olive-green or green in color, with dark green aging, separated by 1-2 concentric circles (zones) of sterile light mycelium. Aerial mycelium abundant, often in the form of hyphal cords. Conidiophores are usually once, sometimes twice branched, coarse, with a rough or dotted shell of 50-125 µ length, with chains of conidia connected by mucus in a dense column up to 150 µ length. Primary twigs 15-20 = 3.5-4 µ; Methili 15-25 µ, sterigma 10-20 µ length. Conidia are elliptical, smooth, pale or dark green 4-7.5 = 3-4 µ.

### Physiological and biochemical properties:

With growth on the environment of Čapek, from carbon sources it absorbs glucose, sucrose, maltose, mannose, galactose, lactose, raffinose, arabinose, sorbose, starch, glycerin. From nitrogen sources, it uses Na nitrate and K nitrate, ammonium sulphate, ammonium nitrate, ammonium chloride, ammonium phosphate monosubstituted, peptone, leucine, lysine, asparagine.

### Strain synthesizes:

Antibiotics: gliokladin, glyoverin, viridin,; Enzymes: Beta-1,2-gluconase, cellobiasis, chitinase; amino acids: glutamine, glycine, arginine, proline, cysteine, meionin, ornithine, lysine, phenylalanine; auxins: indolyl-milk, indol-acetic, indolylcarboxylic acids; hiberryline and abscisic acid; hydrocarbons, including ethylene.

The strain of the fungus Clonostachys rosea f. catenulata VKPM-F1324 is not pathogenic for warm-blooded animals and humans.

Antagonistic properties with respect to a wide range of phytopathogenic fungi and bacteria were investigated. The synthesis of a number of metabolites was also analyzed.

In the course of the experiments, it was found that as a result of the strain, the incidence of plants is significantly reduced, acute forms of damage are removed, the development of plants is accelerated, yield and product quality increase.

The invention is illustrated by the following examples.

Study of the antagonism of the activity of the strain Clonostachys rosea f. catenulata VKPM VKPM-F1324 was compared with the studied Gliocladium strains in Petri dishes and on lawns of phytopathogenic fungi Verticillium dahliae and Fusarium oxysporum. Activity was assessed by the diameter of the zone of no growth of the test object around the antagonist block and by the intensity of the impact of the antagonist on the lawn - the degree of clarification of the lawn, which is marked by pluses.

**Table 1**

| Antagonistic activity of the Clonostachys rosea f catenulata strain VKPM-F1324 and Gliocladium on the nature of interaction with the phytopathogenic fungus Verticillium dahliae on the Chapek's medium | | | |
|---|---|---|---|
| Antagonist fungi | Antagonistic impact radius, cm | Degree of antagonistic effects | Comments |
| Gliocladium roseum 1-27 | 2 | + | + low |
| Gliocladium catenulatum 3 | 2,5 | ++ | ++ medium |
| **Clonostachys rosea f. catenulata VKPM-F1324** | **3** | **+++** | +++ strong |

Evaluation of the protective action of antagonist fungi strains was carried out on gray soil +2 t / ha of lignin in a microvegetation experiment, where Verticillium dahliae microsclerotia 0.15 g / kg were added to the soil along with antagonists. For comparison, the experiment was included strain - Trichoderma B-18. The action of antagonists on Verticillium dahliae in the soil and on cotton infection with verticillus is presented in Table 2.

**Table 2**

| The number of germs of Verticillium dahliae in the soil and the incidence of cotton by Verticillus wilt when entering antagonist fungi into the gray soil with the addition of 2 tons / ha of lignin | | | | |
|---|---|---|---|---|
| Experience Options | The number of KOE of V. dahliae in soil, units / 1 g. | | The infected of cotton,% | Technical efficiency,% |
| | 4 days | 10 days | | |
| Control, soil without antagonist | 4200 | 2100 | 40 | - |
| Gliocladium roseum 1-27 | 3800 | 600 | 10 | 75 |
| Gliocladium catenulatum 3 | 500 | 100 | 10 | 75 |
| Trichoderma harzianum 8-18 | 2900 | 730 | 23 | 42 |
| **Clonostachys rosea f. catenulata VKPM-F1324** | **1000** | **100** | **5** | **85** |

The protective effect of Clonostachys rosea f. catenulata strain VKPM-F1324 was stronger than other strains of Gliocladium catenulatum and Trichoderma.

The phytotoxicity assessment of the culture fluid of the strain was carried out according to the Berestetsky method on the seeds of cucumber and tomato in comparison with other Gliocladium catenulatum 3, Trichoderma harzianum B-18, Fusarium oxysporum and Verticillium dahliae.

**Table 3**

| The effect of filtrates of antagonist fungi and pathogenic fungi on seeds (size of roots, cm) | | | | | | | |
|---|---|---|---|---|---|---|---|
| Medium name | Clear medium | Clonost achysro sea f. ca tenulata VKPM-F1324 | Gliocla dium ca tenulatu m 3 | Gliocladiu m roseum 1-27 | Trichoderm a harzianum 8-18 | Verticilliumd ahliae | Fusarium o xysporum |
| 1 | 2 | 3 | 4 | 5 | 6 | 7 | 8 |
| **Cucumber** | | | | | | | |
| Chapek's | 3,2 | 4,6 | 4,2 | 3,5 | 4 | 2,9 | 2,8 |
| Richard's | 4 | 6,5 | 5,1 | 3,8 | 4,3 | 3 | 2,7 |
| HCP_{0,95} | 0,23 | | | | | | |
| **Tomato** | | | | | | | |
| Chapek's | 1,5 | 2,4 | 2,1 | 1,8 | 2,0 | 1,4 | 1,4 |
| Richard's | 1,5 | 2,8 | 2,3 | 1,8 | 2,1 | 1,2 | 1,3 |
| HCP_{0,95} | 0,15 | | | | | | |

The comparison result indicates a significant stimulating effect on the germination of seeds when soaked in the culture fluid of the strain VKPM-F1324.

Clonostachys rosea f. catenulata strain VKPM-F1324 can also exhibit antibacterial properties. Evaluation of the antibacterial action of the Clonostachys rosea f. catenulata strain VKPM-F1324 was carried out on Petri dishes using the method of opposite colonies with the strain Ralstonia solanacearum. Activity was assessed by the effects of microorganisms on each other. 3 days after the start of the experiment, a contact of the growth zones of the Clonostachys rosea f catenulata strain VKPM-F1324 occurred. and Ralstonia solanacearum bacteria. The growth of the bacteria stopped and the suppression of Ralstonia solanacearum began. After 14 days, the strain of the bacterium Ralstonia solanacearum was completely suppressed by the strain Clonostachys rosea f. catenulata VKPM-F1324.

Thus, the strain Clonostachys rosea f. catenulata deposited in the All-Russian Collection of Industrial Microorganisms of the Federal State Unitary Enterprise GosNIIGenetika on May 18, 2016 under the registration number VKPM-F1324 can be successfully and effectively used against pathogens of fungal and bacterial plant diseases, as well as to stimulate plant growth.

Analysis of phytohormones (auxins, gibberellins, abscisic acid) in the culture fluid of the fungus. For the extraction of auxins, gibberellins and abscisic acid, 20 ml of culture liquid or sterile nutrient medium was taken (control). The solutions were adjusted to pH = 3.0 with 4N hydrochloric acid and extracted with equivalent volumes of ethyl acetate. The obtained extracts were evaporated to dryness under vacuum at 350 ° C and dissolved in 350 µl of 18% acetonitrile. The analysis of auxins in the obtained samples was carried out using the Ultra Performance Liquid Chromatography (LTPLC) method on a Waters ACQUITY UPLC H-class system (Waters, USA). Auxin content analysis was performed using a fluorescence detector at wavelengths: Ex = 280 nm, Em = 350 nm. The analysis was performed on a Waters ACQUITY UPLC BEH RP18 Shield chromatographic column 1.7 µm x 50 mm in an 18% acetonitrile solution with 0.1% acetic acid added at a flow rate of 0.3 ml / min. After analyzing each sample, the column was washed for 3 minutes with 80% acetonitrile. The analysis of abscisic and gibberellic acids was carried out by the same method, but with the detection of the target metabolite on an ultraviolet diode-matrix detector at a wavelength of 265 nm (abscisic acid) and 208 nm (gibberellins).

The results of the analysis are presented in table 4.
Table 4. Biosynthesis of auxins, abscisic and gibberellic acids, ng / ml
Indolyl lactic acid - 32.0
Indolylcarboxylic acid -4.1
IAI-103.4
Abscisic acid-69.5
Gibberellic acid -322,4

### Analysis of the content of amino acids in culture fluids.

Equal volumes of culture liquids (50 ml each) were concentrated under vacuum on a rotary evaporator to a volume of 3 ml. To determine the composition of amino acids, the modern highly sensitive method AccQ-Tag (Waters, USA) was used, based on the use of ACQ derivatizing reagent - 6-aminoquinolyl-N-hydrozysuccinimidyl carbamate, which turns amino acids into stable fluorescent derivatives. Derived derivatives were separated by reverse phase chromatography on a C18 Waters AccQ-Tag column with detection on a fluorescent detector according to the standard Waters method. The content of L-tryptophan was determined in the same concentrated samples, but without treatment with ACQ reagent with detection on a fluorescent detector (fluorescence excitation wavelength - 280 nm, emission wavelength - 350 nm).

The number of detected amino acids is presented in table 5. It is shown that the culture fluid of the fungus is characterized by an increased amount of glutamic acid and glycine.

**Table 5 The content of amino acids, µg / ml.**

| **Aminoacid** | **Sterile medium** | **Fungi** |
|---|---|---|
| Asparaginous | **1,24** | **0,19** |
| Serine | **2,82** | **1,00** |
| Glutamine | **1,33** | **3,30** |
| Glycine | **0** | **2,44** |
| Histidine | **3,76** | **0,49** |
| Arginine | **0** | **0,33** |
| Threonine | **13,10** | **0,71** |
| Alanine | **2,52** | **1,42** |
| Proline | **0,41** | **0,52** |
| GABA | **2,45** | **0,16** |
| Cysteine | **0,04** | **0,25** |
| Tyrosine | **4,61** | **1,16** |
| Valin | **1,82** | **1,02** |
| Methionine | **0,10** | **0,13** |
| Omithine | **0,01** | **0,14** |
| Lysine | **0,14** | **0,92** |
| Isoleucine | **3,34** | **0,87** |
| Leucine | **3,01** | **1,36** |
| Phenylalanine | **0,80** | **1,16** |
| Tryptophan | **0,18** | **0,03** |

| | | |
|---|---|---|
| Control - sterile nutrient medium. GABA - gamma-aminobutyric acid | | |

## Claims

1. Strain Clonostachys rosea f. catenulata deposited in the All-Russian Collection of Industrial Microorganisms under the number VKPM-F1324, used to protect plants against various diseases caused by phytopathogenic fungi and bacteria, as well as a plant growth promoter and producer of metabolites, such as phytohormones and amino acids.

## Patentansprüche

1. Stamm *Clonostachys rosea f. catenulata,* hinterlegt in der Allrussischen Sammlung Industrieller Mikroorganismen unter der Nummer VKPM-F 1324, dient zum Schutz von Pflanzen vor verschiedenen Krankheiten, die durch phytopathogene Pilze und Bakterien verursacht werden, sowie als ein Pflanzenwachstumsförderer und Produzent von Metaboliten wie Phytohormone und Aminosäuren.

## Revendications

1. Souche *Clonostachys rosea f. catenulata* déposée dans la Collection panrusse de micro-organismes industriels sous le numéro VKPM-F1324, utilisée pour protéger les plantes contre diverses maladies causées par des mycètes et des bactéries phytopathogènes, ainsi que comme promoteur de croissance des plantes et producteur de métabolites, tels que des phytohormones et des acides.
